(19) Europäisches Patentamt / European Patent Office / Office européen des brevets

(11) **EP 4 250 308 A1**

(12) **EUROPEAN PATENT APPLICATION**

(43) Date of publication:
**27.09.2023 Bulletin 2023/39**

(21) Application number: **22164338.0**

(22) Date of filing: **25.03.2022**

(51) International Patent Classification (IPC):
**G16H 20/00** (2018.01)   **G16H 50/50** (2018.01)
**G16H 50/20** (2018.01)   **A61M 1/28** (2006.01)

(52) Cooperative Patent Classification (CPC):
**G16H 20/00; A61B 5/4875; A61M 1/1603;**
**A61M 1/1613; G16H 50/20; G16H 50/50;**
A61M 1/1611; A61M 1/28; A61M 2230/08;
A61M 2230/20; A61M 2230/201; A61M 2230/207;
A61M 2230/30

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Fresenius Medical Care Deutschland GmbH**
**61352 Bad Homburg (DE)**

(72) Inventors:
• **Gervasoni, Federica**
  **61352 Bad Homburg (DE)**
• **Neri, Luca**
  **61352 Bad Homburg (DE)**
• **Bellocchio, Francesco**
  **61352 Bad Homburg (DE)**
• **Kuss, Matthias**
  **61352 Bad Homburg (DE)**

(74) Representative: **Bobbert & Partner**
**Patentanwälte PartmbB**
**Postfach 1252**
**85422 Erding (DE)**

(54) **HYDRATION MANAGING SYSTEM**

(57)    The present invention relates to a computer-implemented method for use in assessing a physiological state, in particular the hydration state, of a patient, encompassing the steps of reading in, from a first data source (200), at least one value of at least one first parameter of the patient. Further receiving, from a second data source (210, 220), at least one value of at least one second parameter, the second parameter being related to the patient or to a medical treatment of the latter. Moreover, as a further step providing a database (250) comprising a multitude of combinations of values of a multitude of parameters, gained from said patient and/or from a multitude of patients of a reference patient group. Further, providing a mathematical model for evaluating the at least one read-in value based on at least one of said multitude of combinations of parameter values comprised by the clinical database is also encompassed by the method. Moreover, evaluating the at least one read-in value of the at least one first physiological parameter using the mathematical model and based on the result of said evaluating step outputting a prediction of a future development of at least one physiological state of the patient or at least one of the parameters of the patient, or suggesting future behavior of or treatment for the patient.

Fig. 1

**Description**

The present invention relates to a computer-implemented method for use in assessing a physiological state, in particular the hydration state, of a patient according to claim 1, a system according to claim 12 and devices according to claim 14 or claim 15 or according to the preambles or generic terms of each of these claims.

Returning the patient to a normal hydration state (HD), also referred to as the absence of both overhydration and hypohydration, remains one of the main clinical challenges in dialysis treatments. Indeed, only a minority of patients reaches the target "dry-weight" after the removal of prescribed body fluid volume during dialysis. Abnormal hydration state is associated with a plethora of complications and comorbidity such as arterial hypertension, arterial stiffness, intradialytic hypotensive (IDH) events, pulmonary and peripheral edema, heart failure and increased mortality.

Current challenges in the field include active patients' engagement in fluid management (i.e. including both adherence to fluid and salt intake restriction coupled with physical exercise activation), a lack of reliable instruments for continuous hydration monitoring and/or a complex pathophysiology of complications due to hydration imbalances.

The primary hallmark of advanced chronic kidney disease (CKD) is the inability of kidneys to maintain fluid homeostasis by eliminating excess water. Fluid overload ultimately leads to severe complications including peripheral edema, pulmonary congestion, pleural effusions, hypertension as well as heart failure and increased mortality. Therefore, the primary aim of dialysis treatment is re-establishing fluid homeostasis by replacing insufficient kidney function.

Therefore, an object of the present invention may be to suggest a computer-implemented method for use in assessing a physiological state, in particular the hydration state or its complications such as hypotension, of a patient. Moreover, a system comprising an interdialytic and/or intradialytic monitor should be suggested.

The objective of the present invention may be achieved by the computer-implemented method for use in assessing a physiological state, in particular the hydration state, of a patient having the features of claim 1, by a system having the features of claim 12 and by devices having the features of claim 14 or claim 15.

According to the present invention, a computer-implemented method for assessing or for use in assessing a physiological state, in particular the hydration state, of a patient is suggested.

The method encompasses the step of reading in at least one value of at least one first parameter of the patient from a first data source. The at least one first parameter is a physiological parameter of the patient.

The method encompasses as a further step receiving at least one value of at least one second parameter from a second data source. The first data source and the second data source are optionally at least partly different from each other. The second parameter is also related to the patient, or to a medical treatment of the latter, or to a treatment apparatus with which the patient has been treated or diagnosed, e.g. in the past.

Whenever the term "value" is mentioned herein, this can be understood as single, e.g. discrete, value, a value range and/or the continuous development of a value. A value is a qualitative or quantitative occurrence of the parameter at a given time, also referred to as parameter value. For example, while a parameter is something that can be measured such like the heartbeat, and while measuring it may result in differing, e. g. numerical, values, the value, once measured is a given data, e. g. 64 beats per minute.

Values may be collected with respect to parameters that describe the patient's whole body, at least a segment thereof or with respect to a patient's body layer (skin, tissue or the like), a function of the patient or a state of the patient. Values may also be collected examining or monitoring machine parameters or behavior.

Providing a clinical database which comprises a multitude of combinations of values of a multitude of parameters is also encompassed by the method according to the present invention. Those parameters may include the first parameter and the second parameter. Moreover, the multitude of values were gained from said specific patient and/or from a multitude of patients, preferably of a reference patient group, or their treatments.

The method also encompasses providing a mathematical model for evaluating the at least one read-in value. The evaluation is based on at least one of the said multitude of said combinations of parameter values comprised by the database.

Evaluating the at least one read-in value of the at least one first physiological parameter is also encompassed by the method. For this evaluating step the mathematical model is used. The mathematical model is based on at least one of said combinations of parameter values while at least one of said multitude of combinations of parameter values is retrieved. In this, the combination or combinations retrieved from the clinical database may include the value of the received at least second parameter.

Further encompassed by the method is making and outputting a prediction of a future development of at least one physiological state of the patient and/or outputting at least one of the parameter values, in particular of the first and/or second parameter. The prediction may be based on the result of said evaluating step.

Alternatively or additionally to outputting a prediction and/or the result of the evaluating step, a suggestion concerning the future behavior of or treatment for the patient may also communicated to the patient, medical staff, caregiver and/or doctor. This may be encompassed by the method as a further step.

According to the present invention, a system is suggested.

The system comprises a first data source and at least one second data source, wherein the first data source and the second data source are optionally at least partly different from each other.

Moreover, the system comprises a reading device for reading in at least one value of at least one first parameter of the patient from the first data source. The first parameter is a physiological parameter of the patient.

A receiving device for receiving at least one value of at least one second parameter from the second data source is also comprised by the system. The second parameter is related to the patient or to a medical treatment of the latter.

The system comprises further a clinical database. In this database a multitude of combinations of values of a multitude of parameters are stored and/or provided. These parameters may include the first parameter and the second parameter, wherein the multitude of values were gained from said patient and/or from a multitude of patients of a reference patient group.

A mathematical model is further comprised by the system. The mathematical model serves to evaluate the at least one read-in value based on at least one of said multitude of combinations of parameter values comprised by the clinical database.

Furthermore, the system comprises an evaluating device. The evaluating device serves to evaluate the at least one read-in value of the at least one first physiological parameter using the mathematical model. Therein, the mathematical model is based on at least one of said combinations of parameter values while retrieving at least one of said multitude of combinations of parameter values. The combination or combinations retrieved from the clinical database may include the value of the received at least second parameter.

Based on the result of said evaluating step, an output device that is further comprised by the system, serves to output prediction of a future development of at least one physiological state of the patient or at least one of the parameters of the patient, or to suggest future behavior of or treatment for the patient. The output may be, for example, intended for the patient, caregivers or doctors.

According to the present invention, a device, in particular a hand-held one, is suggested. Such a device may be, e.g., a tablet, a smartphone or the like. The device comprises a control device programmed for executing or triggering the execution of the method according to the present invention.

According to the present invention, a device, in particular a hand-held one, is suggested that comprises two interfaces. One of these interfaces is programmed or configured to read in, from a first data source, at least one value of at least one first parameter of the patient, the other is programmed or configured to receive, from a second data source, at least one value of at least one second parameter. Herein, the first data source and the second data source are preferably at least partly different from each other.

According to the present invention, embodiments of devices as disclosed above may also comprise features or feature combinations of any device according to the present invention as set forth herein.

Embodiments according to the present invention may comprise one or several of the features mentioned supra and/or in the following in any combination unless the person skilled in the art considers the particular combination to be technically impossible.

Advantageous developments of the present invention are each subject-matter of the dependent claims and embodiments.

In all of the aforementioned and following statements, the use of the expression "may be" or "may have" and so on, is to be understood synonymously with "preferably is" or "preferably has", and so on respectively, and is intended to illustrate an embodiment according to the present invention.

Whenever alternatives with "and/or" are introduced herein, the person skilled in the art will understand the "or" contained therein as preferably "either or" and preferably not as "and".

Whenever numerical words are mentioned herein, the person skilled in the art shall recognize or understand them as indications of numerical lower limits. Hence, unless this leads to a contradiction evident for the person skilled in the art, the person skilled in the art shall comprehend for example "one" (or "a/an") as encompassing "at least one". This understanding is also equally encompassed by the present invention as the interpretation that a numerical word, for example, "one" (or "a/an") may alternatively mean "exactly one", wherever this is evidently technically possible in the view of the person skilled in the art. Both of these understandings are encompassed by the present invention and apply herein to all used numerical words.

Whenever an embodiment is mentioned herein, it represents an exemplary embodiment according to the present invention.

When it is disclosed herein that the subject-matter according to the present invention comprises one or several features in a certain embodiment, it is also respectively disclosed herein that the subject-matter according to the present invention does, in other embodiments, likewise according to the present invention, explicitly not comprise this or these features, for example, in the sense of a disclaimer. Therefore, for every embodiment mentioned herein it applies that the converse embodiment, e.g. formulated as negation, is also disclosed.

When method steps are mentioned herein, then the system according to the present invention, which may comprise a calculation device, a control device, a closed-loop control device or the like for this purpose, is in several embodiments configured in order to execute, in any combination, one or several of these method steps, in particular when these are automatically executable steps, or in order to accordingly control corresponding devices which preferably correspond with their names to the designation of the respective method step (for example, "determining" as a method step and "device for determining" for the device) and which may likewise be part of the device(s) according to the present invention or connected thereto in signal communication.

When programmed or configured is mentioned herein, then these terms may in some embodiments be interchangeable.

When a signal communication or communication connection between two components is mentioned herein, this may be understood to mean a connection that exits during use. It may also be understood that a preparation for such a (wired, wireless or otherwise implemented) signal communication exists, for example by coupling both components, for example by pairing, etc.

Pairing is a process that takes place in connection with computer networks in order to establish an initial link between computer units for the purpose of communication. The best-known example of this is the establishing of a Bluetooth connection, by which various devices (e.g. smartphone, headphones) are connected to each other. Pairing is sometimes also referred to as bonding.

When method steps are mentioned herein, then it is provided in several embodiments according to the present invention that these steps are carried out prior to or after a treatment of the patient, for instance during the time when the patient is not connected, e.g. via the extracorporeal blood circuit or the like, to the apparatus or that a treatment has not begun yet or is already completed.

The treatment of the patient may at least be completed or may not have started yet, when, e.g., the patient is not connected to the blood treatment apparatus, e.g., because their vascular system is not connected to an extracorporeal blood circuit in fluid communication, or when no blood or no liquid is returned into their vascular system, in particular not at the relevant point in time and/or not anymore in the further course of the relevant treatment session.

The system may, e. g. via a control device or closed-loop control device or the like, prompt the execution of all or substantially all of the method steps. The method according to the present invention may substantially or completely be executed by, e.g., the control device or closed-loop control device. It may be partly executed by the control device or closed-loop control device. Such method steps may optionally be restricted to machine-induced or automatic method steps.

The term "patient monitor" as used herein may be any device, in particular hand-held or carried-along item (such as smart-watch, general microchip, subcutaneous microchip, band, bracelet, ring, etc.), sensor, software or the like able to collect information about the state (symptoms, physiological measurement, etc.) and the behavior of a specific patient (diet, psycho-physical state, etc.), herein referred to as values of a parameter. In some embodiments, the patient monitor may be in a signal connection to the clinical database or prepared to such a connection. In certain embodiments, the information collected may be sent, at least in part, to the clinical database and stored in there.

In some embodiments of the computer-implemented method, the first data source is or comprises a patient monitor or an interdialytic measuring device.

In several embodiments, the second data source is or comprises a patient monitor, an intradialytic measuring device, a blood treatment apparatus and/or a reporting tool for receiving input by the patient and/or medical staff. The input can be made via touchscreen, keyboard, gesture, language control or in any other way.

In some embodiments, the value(s) of the first and/or the second parameter(s) is/are measured and/or is/are being measured by a wearable bioimpedance sensor, a photoplethysmograph, an accelerometer, a blood glucose sensor, a haemoglobin sensor, a sweat sodium (Na) and/or potassium (K) sensor, a calcium (Ca) sensor, a pulsometer, a skin conductance sensor and/or an actigraph. Those items can form part of the first and/or the second data source.

In several embodiments, the first and/or the second parameter(s) encompass(es) one or more parameter(s) from the group consisting of

- sodium and potassium intake
- sodium muscle content
- drinking behavior pattern
- water intake
- water loss through perspiration and sweat
- water balance
- lean tissue index (LTI)
- fat tissue index (FAT)
- intracellular water (ICW)
- extracellular water (ECW)
- hydration state

- heart rate
- energy intake
- energy consumption
- sleep quality and duration
- physical activity.

In some embodiments, the at least one second parameter encompasses one or more parameter(s) from a group consisting of, or including, data, preferably retrieved from a blood treatment apparatus as second data source during dialytic sessions, including blood pressure dynamics, heart rate dynamics, blood electrolyte profiles (Na, K, Ca), dialysate electrolyte composition (Na, K, Ca), fluid state and/or administered medication, preferably retrieved from a blood treatment apparatus, a patient monitor and/or a clinical database as second data source.

In several embodiments, the multitude of combinations of values provided in the clinical database encompasses or consists of arbitrary combinations of values of parameters of the groups mentioned herein.

In some embodiments, the second parameter(s) and/or the parameters retrieved from the clinical database encompass or consist of one or more parameters from a group consisting of clinical parameters, in particular age, sex, comorbidities, biochemical parameters, drugs, intradialytic events history, data gathered from electronic chart records, patients' symptoms, behavioral attitudes, beliefs and intradialytic and/or interdialytic events assessed with computer adaptive testing applications for self-reported outcomes on patients' smartphones or tablets.

In several embodiments, the output prediction of a future development of at least one physiological state of the patient or at least one of the parameters, which can be in certain embodiments the first physiological parameter, of the patient, or suggestion of a future behavior of or treatment for the patient refers to

- intradialytic hypotension (IDH), in particular if, when or how likely a hypotensive episode may occur;
- maximal tolerable ultrafiltration (UF) rate;
- interdialytic weight gain; and/or
- pre-dialysis systolic and diastolic blood pressure.

In some embodiments, the step of suggesting future behavior of or treatment for the patient, which may be based on predictions, is or encompasses suggesting a particular water and salt intake in an interdialytic period.

In several embodiments, the step of suggesting future behavior or treatment encompasses or consists of individualized behavioral alerts, nudges and health education modules. Hence, the patient and/or, less frequently, the medical staff may receive an alarm, e. g. via the patient monitor, the first data source, a carry-along device such as their wrist watch, mobile, smart phone, smart-watch, or the like. In some embodiments the patient or the medical staff may be provided with medical information such as the newest guidelines on alimentation, water intake management, auxiliary measures and the like.

In some embodiments, outputting or delivering the suggestions to the patient or medical staff and/or making them available to the patient or the medical staff may be achieved, e. g., through a web application, for example, adapted for smartphones and tablets.

In several embodiments, the step of suggesting future behavior encompasses water intake suggestions or recommendations for the patient.

In some embodiments, the accuracy of the retrieved combination of the multitude of parameter values related to the patient is being assessed with regard to the predicted value. It may subsequently be optimized and stored as an optimized version, in particular stored in the clinical database.

In several embodiments, the suggested future behavior of or treatment for the patient is being stored or collected in the clinical database.

In some embodiments, the method according to the present invention comprises the step of calibrating or optimizing the mathematical model.

In several embodiments, the mathematical model is calibrated or optimized using the output prediction or suggestion. For example, the output prediction may come true as predicted, or less so. Hence, monitoring the predicted parameter over time may help optimizing the mathematical model by adapting its weighting of individual parameters, adapting variables or constant used in the calculation of the predicted value, and so on. Artificial intelligence may be helpful here.

In some embodiments, the measurements by the patient monitor, included but not limited to fluid state, blood pressure and heart rate, might be re-calibrated, if needed, using gold-standard measures applying sophisticated analytic techniques, such as artificial intelligence (AI), neural networks, cutting-edge algorithms, mathematical functions and integral transformations.

In several embodiments, the output prediction is evaluated in an additional evaluating step using a measurement of the predicted parameter or state. Tis evaluating step happens in particular in the future.

In some embodiments, the prediction is directed to a particular time point or time window in the future. The evaluating measurement may then take place at this time point or time window.

In several embodiments, the mathematical model is calibrated or optimized using the result of the additional evaluating step. The calibration or optimization may optionally encompass to change the weight of several parameters within the mathematical model.

In some embodiments, the computer-implemented method according to the present invention comprises the step of calibrating or optimizing the multitude of combinations of values of a multitude of parameters on or in the clinical database from a multitude of patients of a reference patient group based on the evaluating steps described herein.

In the clinical database, datasets may be stored which display a (predetermined or previously chosen) accuracy with regard to the patient's predicted parameter. This accuracy may be measured or determined based on a measurement by a reference sensor or reference method. Once the method according to the present invention has been used over sufficient time, these stored datasets will allow a determination or prediction of the parameter in question with an enhanced accuracy, therefore, values predicted by the mathematical model based on such an enhanced database may be included themselves in the clinical database for further use.

In several embodiments, the suggested future treatment of the patient is or encompasses suggesting or requesting for treatment parameters, e.g. ultrafiltration volumes etc., to be accomplished.

In some embodiments, suggestions for treatment parameters may be sent directly via a signal communication to a blood treatment apparatus in order to be used by the latter or a control device thereof for the execution of the next blood treatment session.

In several embodiments, the suggested future behavior of or treatment for the patient is or encompasses making an appointment for the patient or setting a time frame for the next treatment session for the patient to take place.

In some embodiments, the computer-implemented method according to the present invention encompasses the step of correcting the parameter value measured or provided by the first data source by a measurement of said parameter via the second data source.

In several embodiments, the principles of the present invention may be used without a blood treatment apparatus, e.g. in patients suffering from kidney disease who are not yet subject to dialysis.

In some embodiments, the second data source is not a blood treatment apparatus.

In several embodiments, the second data source of the system according to the present invention is or comprises a patient monitor, in particular a smartphone or a smart watch and/or a wearable.

Some or all of the embodiments according to the present invention may comprise one, several or all of the advantages mentioned above and/or in the following.

Complications during hemodialysis (HD) sessions remain one of the main challenges in the dialytic patients' management. Intradialytic hypotension (IDH) is among the most frequent adverse events in HD treatments. The IDH incidence varies widely across studies from less than 10% up to almost 70% of HD sessions, because of the lack of a univocal IDH definition. Inadequate IDH management can lead to adverse outcome including increased mortality risk, hospitalization and vascular access thrombosis. This risk may advantageously be minimized or eliminated by the present invention.

Even patients on Peritoneal Dialysis (PD), which theoretically allows more flexibility in treatment schedules, are not free of overhydration-related complications. Indeed, a large share of PD patients suffers from symptomatic fluid retention. When overhydration is experienced, the hospitalization rate is much higher than that observed for the total PD population. Patient noncompliance with dietary restrictions and with the prescribed PD prescription was identified as the major predictor of fluid retention in several observational studies.

Although several therapeutic algorithms have been proposed for both in-center and home dialysis, appropriate fluid management remains challenging. The possibility of empowering patient's self-care while monitoring dietary patterns and fluid state during the interdialytic period based on real-time patient behaviors monitoring, sensor measurements, and data-driven AI models predicting trajectories of physiological parameters and suggesting optimal therapeutic strategies, may significantly improve patient's outcomes.

Hence, a possible advantage of the present invention may be that it offers an intensified strategy for IDH prevention by predicting the occurrence of these events as one example of a physiological state, or the development of that state, of the patient. Here, by using an AI-based classification model to predict the risk of IDH in the near future, particularly in the next HD treatment, is advantageously implemented.

By a feature selection strategy based on a tradeoff between prediction accuracy and model complexity, a suitable set of input variables may be identified, e.g. including 35 variables. All the variables can be ones that are routinely collected in clinical practice, or they may automatically be recorded from a blood treatment apparatus, therefore, no additional effort may be required by the clinical staff to use the present invention.

A further advantage of the present invention may be that based on the evaluating step forming part of the method according to the present invention, a new or adjusted personalized digital therapeutic program may be installed that may prevent IDH in the specific patient.

Bioimpedance spectroscopy (BIS) techniques, such as BCM (body composition monitor such as the Body Composition Monitor distributed by Fresenius Medical Care, Germany), are non-invasive and accurate methods that measure the body impedance over many electrical frequencies and estimate hydration state values fitting a model function. Moreover, BCM helps to investigate the link between fluid state and blood pressure. Despite the great improvement in HS control through BCM technique, the limit of this approach is the lack of continuative measure since it is time consuming, requires specific equipment and can be used only by trained professionals before a dialysis session. Therefore, fluid dynamics and intake during the interdialytic period is not monitored. The possibility to constantly monitor not only the patient's hydration state but also vital signs and habits by the present invention may advantageously consistently improve complication management and the quality of the patient's life.

The present invention enables the integration of continuous hydration monitoring in a Clinical Decision Support System (CDSS) designed to advantageously activate patients' self-care potential in hydration management and advantageously enable doctors to personalize dialysis prescription for Dialysis Dependent CKD (DD-CKD) and Advanced Non-Dialysis Dependent CKD (stage 5, NDD-CKD).

Due to the monitoring and decision support system included in the present invention, hydration management for patients on DD-CKD and Stage 5 NDD-CKD patients (i.e., drug dose suggestion, dietary suggestion, behavioral change intervention, etc.) may be advantageously optimized in order to be tailored on the specific patient.

The framework of enhancing continuous monitoring measurements (through wearable device) with clinical information may advantageously be applied to a plethora of clinical issues with different nature, such as drugs adaptive administration and chronic disease management.

All the advantages achievable with the method according to the present invention can be in certain embodiments achieved undiminished by the devices, apparatuses or systems according to the present invention and vice versa.

In the following, the present invention is described based on preferred embodiments thereof with reference to accompanying drawing. However, the present invention is not to be limited to these embodiments. In the figures, in which same reference numerals denote identical or similar elements, values and so on, the following applies:

**Fig. 1**   shows a system according to the present invention in a first embodiment; and

**Fig. 2**   shows an exemplary workflow of the method according to the present invention.

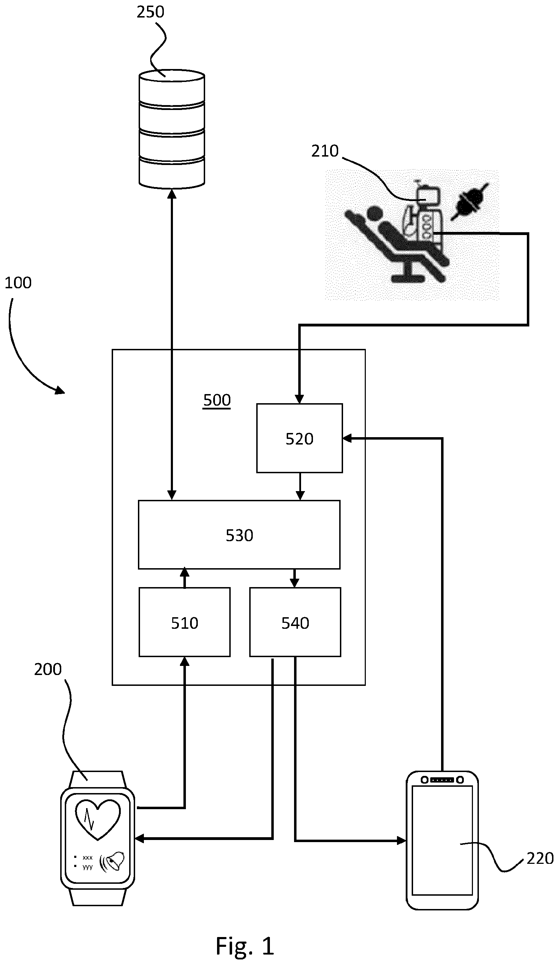

**Fig. 1** shows a system 100 according to the present invention in a first embodiment.

The management of a patient's hydration state (HS) optimally encompasses constant (24h/7d) patient monitoring during both intradialytic sessions and interdialytic periods. The following examples relate to such continuous interdialytic fluid monitoring and management.

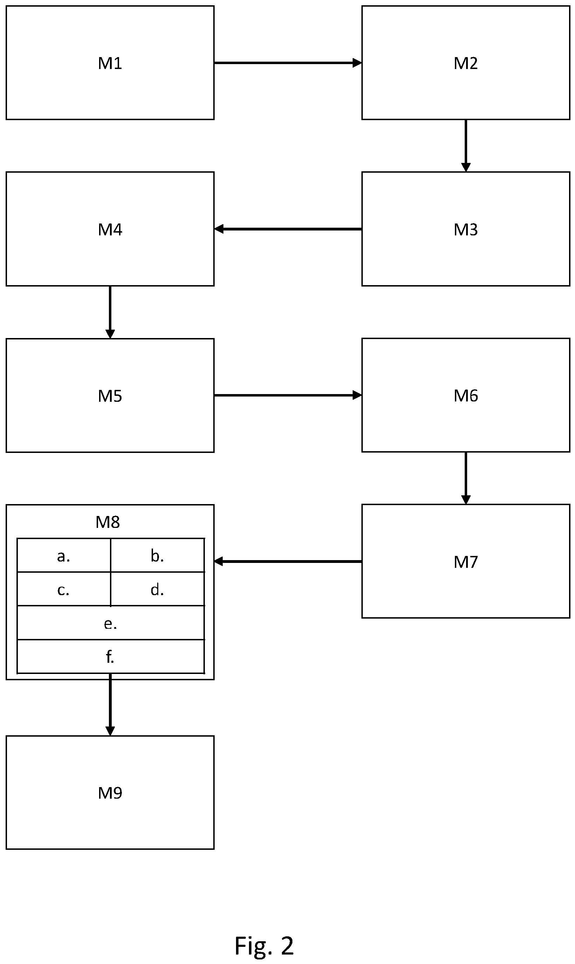

The interdialytic and/or intradialytic monitoring values which may be used as a basis for the method as described with regard to Fig. 2 is accomplished by integrating the information collected from several, partly optional, data sources. The transfer of data or information is represented by arrows, respectively.

In the example of Fig. 1, a first data source is embodied as a patient's smart watch 200. It may capture and/or collect interdialytically and intradialytically values of the following parameters:

1. sodium and potassium intake
2. sodium muscle content
3. drinking behavior pattern
4. water intake
5. water loss through perspiration and sweat
6. water balance
7. lean tissue index (LTI)
8. fat tissue index (FAT)
9. intracellular water (ICW)
10. extracellular water (ECW)
11. hydration state
12. heart rate
13. energy intake
14. energy consumption
15. sleep quality and duration
16. physical activity

In general, the values gained using the first data source may be collected with respect to the patient's whole body, at least a segment thereof or with respect to a patient's body layer (skin, tissue or the like).

Alternatively or additionally, information may be collected by a second data source, in the example of Fig. 1 embodied as a blood treatment apparatus 210, e.g. during an intradialytic session. This information may include, for example, values from the body composition monitor (BCM), vital signs, laboratory tests and results, administered medication/drugs, blood pressure dynamics, heart rate dynamics, blood electrolyte profiles (e.g. Na, K, Ca), dialysate electrolyte composition (e.g. Na, K, Ca) and fluid state. This information may serve to be compared to the information from the first data source 200, to complete this information or to calibrate the latter.

Alternatively or additionally, information may be collected by a reporting tool 220 as a second data source, e.g. the patient's smart phone or tablet. It may in particular serve to capture personal beliefs and impressions, for example (but not limited to) pain, nausea, perspiration, perceived cold or heat, etc. This may, alternatively or additionally, apply for patient's symptoms, behavioral attitudes and/or interdialytic events, which may be assessed with computer adaptive testing applications for self-reported outcomes on patients' smartphones 220 or tablets as reporting tool or as a further data source. This information may again serve to complete the information from the first data source or to calibrate the latter.

Alternatively or additionally, data may be retrieved, for example, from a clinical database 250, where e.g. patient histories, courses of treatments, patient parameters, collective data and the like may be collected and stored. Moreover, clinical parameters, including but not limited to age, sex, comorbidities, biochemical parameters, drugs, intradialytic events history, may be gathered from electronic chart records.

The first and/or second data sources 200, 210, 220, 250 may be or comprise, for example, but are not limited to, wearable bioimpedance sensors, photoplethysmography, accelerometers, blood glucose sensor, hemoglobin sensors, sweat sodium (Na) and potassium (K) sensors, calcium (Ca) sensor, pulsometer, skin conductance sensor and actigraphy and the like.

The values, in particular of the first and/or second parameter, retrieved and collected by the herein-mentioned devices and as described herein, are fed into the calculation device 500 configured for comprising and running a mathematical model, e.g. in an evaluating device 530. Thereby, the values may be or comprise raw values or preprocessed, in particular calibrated, values. As shown in the example of Fig. 1, there may be provided a reading device 510 and a receiving device 520 for this purpose. These devices 510, 520, 530 may be part of the calculation device 500, as shown in Fig. 1, or may be individual devices, possibly in individual housings.

In the embodiment of Fig. 1 the calculation device 500 further comprises an output device 540 or is in signal communication therewith, by which the result achieved by the evaluating device may be processed for output as described below.

In certain embodiments, the first data source 200 and/or the second data source 210, 220 may be prepared and/or suitable for receiving and/or displaying notifications, behavior suggestions or the like. It may be prepared, alternatively or additionally, for issuing an alarm or for reacting to the results of the evaluation of the first parameter as described herein (see Fig. 2). A reaction of a second data source may in particular be or comprise the adjustment of, e.g. treatment parameters, for the specific patient, in particular in their next blood treatment.

**Fig. 2** shows an exemplary workflow of the method according to the present invention. Reference is made to the reference numerals of Fig. 1.

In method step M1, first and second data sources 200, 210, 220, 250 and the calculation device 500 are provided. In this, the calculation device 500 is suitable and/or provided in order to prompt or control the computer-implemented method according to the present invention for use in assessing a physiological state, in particular the hydration state, of a patient.

Method step M2 represents reading in, from a first data source 200, at least one value of at least one first parameter of the patient. Thereby, the first parameter is preferably a physiological parameter of the patient. The at least one value may be a specific value, a value range, a course of the values of a specific parameter taken over time, or the like.

Method step M3 represents receiving, from a second data source 210, 220, at least one value of at least one second parameter, the second parameter being related to the patient or to a medical treatment of the latter. Preferably, such a value is currently measured or otherwise collected and possibly has not (yet) been stored in a database for later reference or use. Thereby, relevant individual and clinical information is collected, e.g. during a dialysis session, e.g. by a sensor of the blood treatment apparatus 210. Moreover, patients can interactively add further information related to relevant events occurred during dialysis session, e.g. via the reporting tool 220.

In some embodiments, such a value may have been measured or collected in the past.

Method step M4 represents providing a clinical database 250 comprising a multitude of combinations of values of a multitude of parameters, those parameters may include the first parameter and the second parameter. Said multitude of values were gained from said patient and/or from a multitude of patients of a reference patient group.

By the method steps M2 to M3 a wide range of interdialytic and intradialytic information regarding the first parameter or the second parameter may be measured or otherwise collected by frequently or even endlessly monitoring the patient, e. g. via the patient monitor or, more general, via the first data source, or frequently monitoring the second parameter, e. g. via the treatment apparatus 210, or, more general, via the second data source. This information may be stored in the clinical database 250 as values.

Method step M5 represents providing a mathematical model for evaluating the at least one read-in value based on at least one of the said multitude of said combinations of parameter values comprised by the clinical database 250. The mathematical model may be executed or hosted by the calculation device 500.

Method step M6 represents evaluating the at least one read-in value of the at least one first physiological parameter using the mathematical model based on at least one of said combinations of parameter values while retrieving at least one of said multitude of combinations of parameter values. The combination or combinations retrieved from the clinical database 250 may include the received value of the at least second parameter without being limited thereto. In fact, the value of the second parameter may have been previously collected and stored in the clinical database 250 before the value of the first parameter was available and, hence, received. However, the value of the second parameter may be measured or collected at about the same time when the value of the first parameter has been selected. Hence, the value of the second parameter may be, at the time of the evaluation, just recently gained and, hence, not have been stored (yet).

Represented by method step M7 in Fig. 2, some or all of the captured and collected values such as the first or second parameter, i.e., physiological and optionally behavioral parameters, may be assessed by using, e.g., signal integration. This may encompass a (re-)calibration of data, if needed, using gold-standard measures applying sophisticated analytic techniques, such as artificial intelligence (AI), neural network, cutting-edge algorithms, mathematical functions and integral transformations.

Step M7 may be based on a formula such as the formula (1):

$$M_{gold\_standard}=f(M_{patient\_monitor},\ individual\_characteristics)+\varepsilon \qquad (1)$$

wherein

| | |
|---|---|
| f | is any function obtained by any analytic methods, including but not limited to machine learning techniques, mathematical approximation techniques, mathematical transformation techniques (such as Laplace transformation, wavelet, ...), cutting-edge algorithms. |
| $M_{gold\_standard}$ | is any relevant gold standard measure. For example, the gold standard measure for hydration state could encompasses different technologies, including, but not limited to, total body water (TBW) by isotope, bioimpedance spectroscopy (BIS) techniques, including but limiting to BCM (body composition monitor), multiple-frequency whole-body (BIA). |
| $M_{patient\_monitor}$ | is any relevant measure, including but not limited to hydration state, blood pressure, heart rate, energy balance, sleep duration and quality, obtained from interdialytic and intradialytic phases using any patient monitor. |
| individual_characteristics | represents a vector of patient-specific information derived from the constant intradialytic and interdialytic monitoring during and between blood treatment sessions using, e.g., the patient monitor device 200 and/or the blood treatment apparatus 210. |
| $\varepsilon$ | is a vector describing the error of the approximation. |

In method step M8, based on the result of the evaluating step described herein, a prediction of a future development of at least one physiological state of the patient or at least one of the parameters, which in some embodiments may also be the first (physiological) parameter, is made. Hence, the evaluation may give a prospect or forecast of how the first parameter may develop in the near or foreseeable future. However, the evaluation may not only predict the future course of the first parameter. Rather the prospect or forecast may, for example, shed light on the question if, and in the affirmative, when for example a hypotension episode must be expected, be it intradialytic (during one treatment session) or interdialytic (between two treatment sessions).

In this method step, for example, the following predictions or forecasts can be made:

    a. Intradialytic hypotension (IDH) and maximal tolerable ultrafiltration (UF) rate;
    b. Interdialytic weight gain;
    c. Pre-dialysis systolic and diastolic blood pressure;
    d. Interdialytic water and salt intake;
    e. Based on predictions in a. to c., maximal tolerable water and salt intake in the interdialytic period may be computed;

Represented by the reference numeral f., individualized behavioral alerts, nudges and health education modules based on behavioral and physiologic function monitoring in the interdialytic period in order to achieve maximal tolerable water and salt intake computed in e. are computed based on the results of the evaluation of the mathematical models and the predictions as described herein.

In method step M9, the features computed in or for step a. to f. are made available to the patient, e.g. by displaying them, for example, on the patient monitor 210, i.e. the patient's smart watch or on the reporting tool 220, i.e. the patient's tablet. In this, individualized behavioral alerts, nudges and health education modules are sent to the patients preferably through a web application optimized for multiple platforms. This web application is, optionally and advantageously, optimized for the patient's smartphone 200 and/or tablet 240 already used with the method according to the present invention.

Alternatively or additionally, the features computed in or for step a. to f. are made available to a caregiver or medic in order to be able to adjust further treatment plans or treatment parameters for the specific patient. This may also encompass suggesting at least one time point for a following treatment session.

In specific embodiments, all or at least part of the data collected by the data sources 200, 210, 220 for intradialytic and/or interdialytic measures may be collected in the clinical database 250. This also applies to the prediction achieved in the steps a. to f. This information in the clinical database 250 may serve to the steady and continuous increase in the accuracy of the mathematical model on which the method according to the present invention is based.

**Reference Numerals**

100    system

200    first data source; e.g. interdialytic or intradialytic measuring device (here exemplarily in the form of a patient's smart watch)

210    second data source; intradialytic measuring device (here exemplarily in the form of a blood treatment apparatus)

220    second data source; reporting tool (here exemplarily in the form of a patient's tablet)

250    clinical database

500    calculation device

510    reading device

520    receiving device

530    evaluating device

540    output device

M1 to M9    method steps

a. to f.    predictions and evaluations

**Claims**

1.  A computer-implemented method for use in assessing a physiological state, in particular the hydration state, of a patient, encompassing the steps:

    - reading in, from a first data source (200), at least one value of at least one first parameter of the patient, the first parameter being a physiological parameter;
    - receiving, from a second data source (210, 220), at least one value of at least one second parameter, the second parameter being related to the patient or to a medical treatment of the latter, the first data source (200) and the second data source (210, 220) being at least partly different from each other;
    - providing a clinical database (250) comprising a multitude of combinations of values of a multitude of parameters, those parameters including the first parameter and the second parameter, wherein the multitude of values were gained from said patient and/or from a multitude of patients of a reference patient group;
    - providing a mathematical model for evaluating the at least one read-in value based on at least one of said multitude of combinations of parameter values comprised by the clinical database;
    - evaluating the at least one read-in value of the at least one first physiological parameter using the mathematical model, the evaluation being based on at least one of said combinations of parameter values while retrieving at least one of said multitude of combinations of parameter values, wherein the combination or combinations retrieved from the clinical database (250) include(s) the value of the received at least one second parameter and optionally also the read-in value of the at least one first parameter; and
    - based on the result of said evaluating step outputting a prediction of a future development of at least one physiological state of the patient or at least one of the parameters of the patient, e.g. the at least one first parameter, or suggesting future behavior or treatment for or of the patient;

wherein the first data source (200) is or comprises a patient monitor or an interdialytic measuring device; and wherein the second data source (210, 220) is or comprises a patient monitor, an intradialytic measuring device, a blood treatment apparatus and/or a reporting tool for receiving input by the patient and/or medical staff.

2. The computer-implemented method according to any one of the preceding claims, wherein the value(s) of the first and/or the second parameters is/are measured and/or is/are being measured by a wearable bioimpedance sensor, a photoplethysmograph, an accelerometer, a blood glucose sensor, a hemoglobin sensor, a sweat sodium (Na) and/or potassium (K) sensor, a calcium (Ca) sensor, a pulsometer, a skin conductance sensor and/or an actigraph.

3. The computer-implemented method according to any one of the preceding claims,

wherein the first and/or the second parameters encompass(es) one or more parameter(s) from the group consisting of

- sodium and potassium intake
- sodium muscle content
- drinking behavior pattern
- water intake
- water loss through perspiration and sweat
- water balance
- lean tissue index (LTI)
- fat tissue index (FAT)
- intracellular water (ICW)
- extracellular water (ECW)
- hydration state
- heart rate
- energy intake
- energy consumption
- sleep quality and duration
- physical activity,

and/or
wherein the at least one second parameter encompasses one or more parameter(s) from a group consisting of, or including, blood pressure dynamics, heart rate dynamics, blood electrolyte profiles (Na, K, Ca), dialysate electrolyte composition (Na, K, Ca), fluid state and/or administered medication, preferably retrieved from a blood treatment apparatus (210), a patient monitor 200 and/or a clinical database (250) as second data source during dialytic sessions.

4. The computer-implemented method according to at least one of the preceding claims,

wherein the second parameter(s) and/or the parameters retrieved from the clinical database (250) encompass or consist of one or more parameters from a group consisting of clinical parameters, in particular age, sex, comorbidities, biochemical parameters, drugs, intradialytic events history, data gathered from electronic chart records, patients' symptoms, behavioral attitudes, beliefs and intradialytic and/or interdialytic events assessed with computer adaptive testing applications for self-reported outcomes on patients' smartphones or tablets; and/or
wherein the output is a prediction or suggestion of future behavior of or treatment for the patient is related to or consist of

- intradialytic hypotension (IDH);
- maximal tolerable ultrafiltration (UF) rate;
- interdialytic weight gain; and/or
- pre-dialysis systolic and diastolic blood pressure.

5. The computer-implemented method according to at least one of the preceding claims, wherein the step of suggesting future behavior of or treatment for the patient is or encompasses suggesting a particular water and salt intake in an interdialytic period.

6. The computer-implemented method according to at least one of the preceding claims, wherein the accuracy of the retrieved combination of the multitude of parameter values related to the patient is being assessed with regard to the predicted value, optimized and stored, in particular in the clinical database (250), as an optimized version.

7. The computer-implemented method according to at least one of the preceding claims, comprising the step of calibrating or optimizing the mathematical model, and/or wherein the mathematical model is calibrated or optimized using the output prediction or suggestion.

8. The computer-implemented method according to at least one of the preceding claims, wherein the measurements from the first data source (200), included but not limited to fluid state, blood pressure and heart rate, might be re-calibrated, if needed, using gold-standard measures applying sophisticated analytic techniques, such as artificial intelligence (AI), neural networks, cutting-edge algorithms, mathematical functions and integral transformations.

9. The computer-implemented method according to any one of the preceding claims,

   wherein the output prediction is evaluated in an additional evaluating step using a measurement of the predicted parameter or state; and/or
   wherein the mathematical model is calibrated or optimized using the result of the additional evaluating step.

10. The computer-implemented method according to any one of the preceding claims, wherein the suggested future treatment of the patient is or encompasses suggesting or requesting treatment parameters, e.g. ultrafiltration volumes, to be accomplished.

11. The computer-implemented method according to any one of the preceding claims, wherein the suggested future behavior of or treatment for the patient is or encompasses ordering patient or setting a time frame for the next treatment session for the patient to take place.

12. A system (100) comprising

   - a first data source (200);
   - a reading device (510) for reading in, from the first data source (200), at least one value of at least one first parameter of the patient, the first parameter being a physiological parameter;
   - a second data source (210, 220), the first data source (200) and the second data source (210, 220) being at least partly different from each other;
   - a receiving device (520) for receiving, from the second data source (210, 220), at least one value of at least one second parameter, the second parameter being related to the patient or to a medical treatment of the latter;
   - a clinical database (250) comprising a multitude of combinations of values of a multitude of parameters, those parameters including the first parameter and the second parameter, wherein the multitude of values were gained from said patient and/or from a multitude of patients of a reference patient group;
   - a mathematical model for evaluating the at least one read-in value based on at least one of said multitude of combinations of parameter values comprised by the clinical database;
   - an evaluating device (530) for evaluating the at least one read-in value of the at least one first physiological parameter using the mathematical model based on at least one of said combinations of parameter values while retrieving at least one of said multitude of combinations of parameter values, wherein the combination or combinations retrieved from the clinical database (250) include(s) the value of the received at least second parameter; and
   - an output device (540) for, based on the result of said evaluating step, outputting a prediction of a future development of at least one physiological state of the patient or at least one of the parameters of the patient, or suggesting future behavior or treatment for or of the patient.

13. The system (100) according to claim 12, wherein the second data source (210, 220) is or comprises a patient monitor, in particular a smartphone or a smart watch and/or a wearable.

14. A device, in particular a hand-held one, comprising a control device programmed for executing or triggering the execution of the method according to any one of claims 1 to 11.

15. A device, in particular a hand-held one, in particular according to claim 14, comprising two interfaces programmed or configured to read in, from a first data source (200), at least one value of at least one first parameter of the patient, and to receive, from a second data source (210, 220), at least one value of at least one second parameter, the first data source (200) and the second data source (210, 220) being at least partly different from each other.

Fig. 1

Fig. 2

# EP 4 250 308 A1

## EUROPEAN SEARCH REPORT

**Application Number**

EP 22 16 4338

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/257718 A1 (MEDICI TECHNOLOGIES LLC) 24 December 2020 (2020-12-24) | 1-3,6-15 | INV.<br>G16H20/00 |
| Y | * paragraph [0024] *<br>* paragraph [0075] – paragraph [0077] *<br>* paragraph [0096] – paragraph [0100] *<br>* paragraph [0138]; figure 10 *<br>* paragraph [0155] – paragraph [0161]; figures 17, 18 *<br>* paragraph [0180] – paragraph [0190]; figures 31-33 *<br>* paragraph [0200] – paragraph [0206] *<br>* figure 20 *<br>* paragraph [0148] * | 4,5 | G16H50/50<br>G16H50/20<br>A61M1/28 |
| X | US 2019/069245 A1 (MILLER DEVIN W [US] ET AL) 28 February 2019 (2019-02-28) | 1-3,6-15 | |
| Y | * paragraph [0125] *<br>* paragraph [0184] *<br>* paragraph [0162] – paragraph [0179] *<br>* figures 20, 21 *<br>* paragraph [0109] *<br>* paragraph [0093] – paragraph [0094] *<br>* paragraph [0155] *<br>* paragraph [0081] *<br>* paragraph [0059] – paragraph [0060] * | 4,5 | |
| X | US 10 485 475 B1 (MILLER DAVID R [US] ET AL) 26 November 2019 (2019-11-26)<br>* column 15, line 1 – line 3 *<br>* column 40, line 54 – column 42, line 5 * | 1-15 | **TECHNICAL FIELDS SEARCHED (IPC)**<br><br>G16H<br>A61M |
| Y | US 2016/038043 A1 (MULLIGAN ISOBEL JANE [US] ET AL) 11 February 2016 (2016-02-11)<br>* paragraph [0072] – paragraph [0097] *<br>* paragraph [0102] – paragraph [0104]; figure 1B *<br>* paragraph [0106] – paragraph [0113] *<br>* paragraph [0136] – paragraph [0137] * | 4,5 | |

-/--

The present search report has been drawn up for all claims

| Place of search | Date of completion of the search | Examiner |
|---|---|---|
| Munich | 21 July 2022 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 22 16 4338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/104989 A1 (BREAUX JAMES [US] ET AL) 11 April 2019 (2019-04-11) <br> * paragraph [0044] – paragraph [0048] * <br> * paragraph [0034] – paragraph [0038] * <br> ----- | 3 | |
| A | EP 3 654 347 A1 (KAUNAS UNIV OF TECHNOLOGY [LT]) 20 May 2020 (2020-05-20) <br> * paragraph [0002] – paragraph [0005] * <br> ----- | 2 | |

TECHNICAL FIELDS
SEARCHED     (IPC)

The present search report has been drawn up for all claims

| Place of search | Date of completion of the search | Examiner |
|---|---|---|
| Munich | 21 July 2022 | Eichenauer, Lars |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 16 4338

This annex lists the patent family members relating to the patent documents cited in the above-mentioned European search report.
The members are as contained in the European Patent Office EDP file on
The European Patent Office is in no way liable for these particulars which are merely given for the purpose of information.

21-07-2022

| Patent document cited in search report | | Publication date | Patent family member(s) | | Publication date |
|---|---|---|---|---|---|
| WO 2020257718 | A1 | 24-12-2020 | CN 114340480 A | | 12-04-2022 |
| | | | EP 3986260 A1 | | 27-04-2022 |
| | | | KR 20220024685 A | | 03-03-2022 |
| | | | US 2022096007 A1 | | 31-03-2022 |
| | | | WO 2020257718 A1 | | 24-12-2020 |
| US 2019069245 | A1 | 28-02-2019 | US 10154460 B1 | | 11-12-2018 |
| | | | US 10307101 B1 | | 04-06-2019 |
| | | | US 10485475 B1 | | 26-11-2019 |
| | | | US 11109805 B1 | | 07-09-2021 |
| | | | US 2019069245 A1 | | 28-02-2019 |
| | | | US 2019246977 A1 | | 15-08-2019 |
| US 10485475 | B1 | 26-11-2019 | US 10154460 B1 | | 11-12-2018 |
| | | | US 10307101 B1 | | 04-06-2019 |
| | | | US 10485475 B1 | | 26-11-2019 |
| | | | US 11109805 B1 | | 07-09-2021 |
| | | | US 2019069245 A1 | | 28-02-2019 |
| | | | US 2019246977 A1 | | 15-08-2019 |
| US 2016038043 | A1 | 11-02-2016 | NONE | | |
| US 2019104989 | A1 | 11-04-2019 | US 2019104989 A1 | | 11-04-2019 |
| | | | US 2022022812 A1 | | 27-01-2022 |
| EP 3654347 | A1 | 20-05-2020 | EP 3654347 A1 | | 20-05-2020 |
| | | | LT 6780 B | | 10-11-2020 |

EPO FORM P0459

For more details about this annex : see Official Journal of the European Patent Office, No. 12/82